# EUROPEAN PATENT APPLICATION

(11) **EP 4 789 624 A1**
(43) Date of publication of application: **12.08.2026**
(21) Application number: 24918223.9
(22) Date of filing: 22.11.2024
(51) Int. Cl.: A61B 17/00, A61B 17/32

(54) **LIQUID PIPE SLEEVE, MEDICAL ULTRASONIC KNIFE, AND ROBOT-ASSISTED ULTRASONIC KNIFE SYSTEM**

(30) Priority: 17.01.2024 CN 202420120428 U
(71) Applicant: SMTP Medical Co., Ltd., Beijing 102629 (CN)
(72) Inventor: CAO, Qun, Beijing 102629 (CN); ZHAN, Songtao, Beijing 102629 (CN); DAI, Zhiling, Beijing 102629 (CN); FANG, Yingfei, Beijing 102629 (CN)
(74) Representative: Reddie & Grose LLP
(86) International application number: PCT/CN2024/133839
(87) International publication number: WO 2025/152614

(57) **Abstract**

Provided are a liquid flow sleeve, a medical ultrasonic scalpel, and a robot-assisted ultrasonic scalpel system. The liquid flow sleeve (100) includes a sleeve body (10), an adjustment assembly (20), a mounting assembly (30), and a connecting assembly (40). The sleeve body (10) extends in a first direction X, and the sleeve body (10) has a first end (11) and a second end (12) arranged opposite each other in the first direction X. The mounting assembly (30) is connected to the first end (11) of the sleeve body (10), and the mounting assembly (30) is configured to connect to the handle (200). The adjustment assembly (20) has a third end (21) and a fourth end (22) arranged opposite each other in the first direction X. The connecting assembly (40) connects the sleeve body (10) and the adjustment assembly (20). The adjustment assembly (20) can move in the first direction X to change a maximum distance between an end portion of the second end (12) and an end portion of the fourth end (22) in the first direction X.

## Description

### CROSS REFERENCE

The present application refers to Chinese Patent Application No. 202420120428.9, filed on January 17, 2024 and entitled "LIQUID FLOW SLEEVE, MEDICAL ULTRASONIC SCALPEL, AND ROBOT-ASSISTED ULTRASONIC SCALPEL SYSTEM", which is incorporated herein by reference in its entirety.

### TECHNICAL FIELD

The present application relates to the technical field of medical devices, and in particular to a liquid flow sleeve, a medical ultrasonic scalpel and a robot-assisted ultrasonic scalpel system.

### BACKGROUND

With the development of ultrasound technology and its integration with modern medicine, medical ultrasonic scalpels have gradually been applied in surgical procedures. A medical ultrasonic scalpel includes a handle, a blade, and a liquid flow sleeve. The blade and the liquid flow sleeve are both connected to one end of the handle. The liquid flow sleeve is sleeved on the blade, and a front end of the blade extends out of the liquid flow sleeve to cut tissue. During the surgical procedure of the medical ultrasonic scalpel, coolant can be delivered to the front end of the blade through the liquid flow sleeve to cool the blade.

In the related art, after the medical ultrasonic scalpel is mounted, the length of the front end of the blade extending beyond the liquid flow sleeve is fixed. If it is necessary to change the length of the front end of the blade extending beyond the liquid flow sleeve, the liquid flow sleeve needs to be removed and replaced with a new liquid flow sleeve. The range of applications of the liquid flow sleeve is relatively narrow.

### SUMMARY

The present application is intended to solve at least one of the technical problems existing in the background art. To this end, an objective of the present application is to provide a liquid flow sleeve, a medical ultrasonic scalpel, and a robot-assisted ultrasonic scalpel system, achieving a wider range of applications of the liquid flow sleeve.

Embodiments in a first aspect of the present application provide a liquid flow sleeve. The liquid flow sleeve includes: a sleeve body extending in a first direction X, the sleeve body having a first end and a second end arranged opposite each other in the first direction X; an adjustment assembly having a third end and a fourth end arranged opposite each other in the first direction X; a mounting assembly connected to the first end of the sleeve body, the mounting assembly being configured to connect to a handle; and a connecting assembly connecting the sleeve body and the adjustment assembly; where the adjustment assembly is movable in the first direction X to change a maximum distance between an end portion of the second end and an end portion of the fourth end in the first direction X.

In some embodiments, the adjustment assembly includes: an adjustment sleeve, an outer side wall of the adjustment sleeve fitting against an inner side wall of the sleeve body, and the third end being located at the adjustment sleeve; and an adjustment portion connected to the adjustment sleeve, the adjustment portion and the third end being located at two opposite ends of the adjustment sleeve in the first direction X, the fourth end being located at an opposite end from the connection of the adjustment portion with the adjustment sleeve, and an outer side wall of the adjustment portion having a plurality of fixing slots spaced in the first direction X; where one end of the connecting assembly is connected to an outer side wall of the sleeve body, and the other end of the connecting assembly has a fixing protrusion movably located in the fixing slot, where the fixing protrusion fits with the fixing slots such that the maximum distance between the end portion of the second end and the end portion of the fourth end in the first direction X is different when the fixing protrusion is located in different fixing slots among the plurality of fixing slots.

In some embodiments, the outer side wall of the sleeve body has a fixing groove, two opposite side walls of the fixing groove each having a first mounting hole; and the connecting assembly includes: a torsion spring located in the fixing groove, one of torsion arms of the torsion spring abutting against a bottom surface of the fixing groove; a pressing snap-fit member, the fixing protrusion being connected to an end portion of the pressing snap-fit member, the other end of the pressing snap-fit member opposite the fixing protrusion having a pressing portion, the pressing snap-fit member having a second mounting hole, and the second mounting hole being located between the fixing protrusion and the pressing portion; and a connecting shaft sequentially passing through one first mounting hole of the two first mounting holes, the second mounting hole, a center hole of the torsion spring, and the other first mounting hole of the two first mounting holes; where the other torsion arm of the torsion spring abuts against a surface of the pressing portion facing the fixing groove.

In some embodiments, each of the fixing slots includes a first face and a second face facing each other, and an angle between the first face and the second face is an acute angle; and the fixing protrusion includes a third face and a fourth face opposite each other, and when the fixing protrusion is located in the fixing slot, the third face fits against the first face, and the fourth face fits against the second face.

In some embodiments, the first face is perpendicular to the first direction X, and for the first face and the second face in the same fixing slot, the second face is closer to the third end, and the first face is closer to the fourth end.

In some embodiments, both the first face and the second face form an angle greater than 30 degrees with the first direction X.

In some embodiments, an extension direction of the connecting shaft is perpendicular to the first direction X.

In some embodiments, the extension direction of the connecting shaft is parallel to the first direction X, and a side wall of the sleeve body has a fixing through hole for the fixing protrusion to extend into an inner cavity of the sleeve body.

In some embodiments, the adjustment portion includes a first arc-shaped adjustment plate, the first arc-shaped adjustment plate extending in the first direction X, one end of the first arc-shaped adjustment plate in the first direction X being connected to the adjustment sleeve, and the other end of the first arc-shaped adjustment plate in the first direction X being a free end, or the adjustment portion includes a connecting tube and a plurality of second arc-shaped adjustment plates, the plurality of second arc-shaped adjustment plates extending in the first direction X, one end of the plurality of second arc-shaped adjustment plates in the first direction X being connected to the adjustment sleeve, the other end of the plurality of second arc-shaped adjustment plates in the first direction X being connected to the connecting tube, the plurality of second arc-shaped adjustment plates being spaced along a circumference of the adjustment sleeve, and the fixing slots being located in at least one of the plurality of second arc-shaped adjustment plates; or the adjustment portion includes a cylindrical tube, the cylindrical tube extending in the first direction X.

In some embodiments, the outer side wall of the adjustment portion has a limiting protrusion, the limiting protrusion and the plurality of fixing slots being arranged in the first direction X, and the limiting protrusion being located on a side of the plurality of fixing slots close to the fourth end.

In some embodiments, the inner side wall of the sleeve body has a first planar face, the first planar face being parallel to the first direction X, and the first planar face extending to an end face of the second end; the outer side wall of the adjustment sleeve has a second planar face, the second planar face extending to an end face of the other end of the adjustment sleeve opposite the third end; and the first planar face fits against the second planar face.

In some embodiments, the inner side wall of the sleeve body has a first boss protruding into the inner cavity of the sleeve body, and a surface of the first boss facing the inner cavity of the sleeve body is the first planar face; in a direction perpendicular to the second planar face, a height H of the other end of the adjustment sleeve opposite the third end is less than a height H of the third end, such that the third end of the adjustment sleeve forms a second boss, the second boss being located on a side of the first boss away from the second end; and an orthographic projection of the first boss on a first surface at least partially overlaps with an orthographic projection of the second boss on the first surface, the first surface being perpendicular to the first direction X.

In some embodiments, the mounting assembly is sleeved on the first end of the sleeve body, and the outer side wall of the sleeve body has a circle of mounting groove at the first end, the mounting groove surrounding the sleeve body along a circumference of the sleeve body; and the mounting assembly has a circle of first annular boss on the inner side wall of an end facing the sleeve body, the first annular boss being located in the mounting groove.

In some embodiments, the outer side wall of the sleeve body has a second annular boss and a third annular boss spaced in the first direction X, and the mounting groove is formed between the second annular boss and the third annular boss.

In some embodiments, one of the inner side wall of the sleeve body and the outer side wall of the adjustment sleeve has a guide groove, the guide groove extending in the first direction X; and the other of the inner side wall of the sleeve body and the outer side wall of the adjustment sleeve has a guide protrusion, the guide protrusion being movably located within the guide groove.

In some embodiments, the adjustment assembly includes a plurality of sub-adjustment assemblies, the plurality of sub-adjustment assemblies being connected by means of the connecting assembly, and any two adjacent sub-adjustment assemblies being movably connected in the first direction X.

A medical ultrasonic scalpel is provided according to embodiments in a second aspect of the present application. The medical ultrasonic scalpel includes: a liquid flow sleeve of any of the embodiments described above; a handle connected to the mounting assembly of the liquid flow sleeve; and a blade connected to the handle, the blade and the liquid flow sleeve being both connected to a same end of the handle, and the liquid flow sleeve being sleeved on the blade.

A robot-assisted ultrasonic scalpel system is provided according to embodiments in a third aspect of the present application. The robot-assisted ultrasonic scalpel system includes a robot-assisted surgical device, and a medical ultrasonic scalpel according to the claims; where the robot-assisted surgical device is connected to a blade of the medical ultrasonic scalpel to control the movement of the blade.

The length of the liquid flow sleeve provided in the embodiments of the present application is positively correlated with the maximum distance between the end portion of the second end and the end portion of the fourth end in the first direction. Once the liquid flow sleeve and the blade are mounted, the length of the blade is fixed, and the length of the liquid flow sleeve can be adjusted by controlling the adjustment assembly to move in the first direction. When the length of the liquid flow sleeve is changed, the extension length of the blade can also be changed. The extension length of the blade can be adjusted by controlling the adjustment assembly to move in the first direction. There is no need to remove the liquid flow sleeve and replace it with a new liquid flow sleeve or blade. The liquid flow sleeve is suitable for more scenarios, and the liquid flow sleeve has a wider range of applications.

### BRIEF DESCRIPTIONS OF THE DRAWINGS

In the accompanying drawings, the same reference signs denote the same or similar components or elements throughout a plurality of accompanying drawings unless otherwise specified. These accompanying drawings are not necessarily drawn to scale. It should be understood that these accompanying drawings depict only some embodiments according to the present application herein and are not to be construed as limiting the scope of the present application.
FIG. 1 shows a schematic structural diagram of a liquid flow sleeve according to an embodiment of the present application;
FIG. 2 shows a schematic exploded view of the liquid flow sleeve according to an embodiment of the present application;
FIG. 3 shows a schematic structural diagram of a sleeve body according to an embodiment of the present application;
FIG. 4 shows a schematic cross-sectional view of the sleeve body according to an embodiment of the present application;
FIG. 5 shows a schematic structural diagram of an adjustment assembly according to an embodiment of the present application;
FIG. 6 shows a schematic cross-sectional view of the liquid flow sleeve according to an embodiment of the present application in one state;
FIG. 7 shows a schematic cross-sectional view of the liquid flow sleeve according to an embodiment of the present application in another state;
FIG. 8 shows a schematic structural diagram of a mounting assembly according to an embodiment of the present application;
FIG. 9 shows a partial schematic cross-sectional view of the adjustment assembly fitting with the sleeve body according to an embodiment of the present application;
FIG. 10 shows a partial enlarged view of part A in FIG. 9;
FIG. 11 shows a partial schematic cross-sectional view of the adjustment assembly fitting with the sleeve body in another state according to an embodiment of the present application;
FIG. 12 shows a partial schematic cross-sectional view of a fixing protrusion fitting with a fixing slot according to an embodiment of the present application;
FIG. 13 shows a schematic structural diagram of a further liquid flow sleeve according to an embodiment of the present application;
FIG. 14 shows a partial schematic exploded view of the further liquid flow sleeve according to an embodiment of the present application;
FIG. 15 shows a schematic cross-sectional view of the further liquid flow sleeve according to an embodiment of the present application;
FIG. 16 shows a schematic cross-sectional view of the further liquid flow sleeve according to an embodiment of the present application in another cross section;
FIG. 17 shows a schematic cross-sectional view of the further liquid flow sleeve according to an embodiment of the present application in another state;
FIG. 18 shows a schematic structural diagram of a further liquid flow sleeve according to an embodiment of the present application;
FIG. 19 shows a schematic structural diagram of a further liquid flow sleeve according to an embodiment of the present application; and
FIG. 20 shows a schematic structural diagram of a medical ultrasonic scalpel according to an embodiment of the present application.

### List of reference signs:

100. Liquid flow sleeve; 200. Handle; 300. Blade;
10. Sleeve body; 11. First end; 12. Second end; 13. Fixing groove; 14. First mounting hole; 15. Fixing through hole; 16. First planar face; 17. First boss; 18. Mounting groove; 19. Second annular boss; 110. Third annular boss; 111. Fourth annular boss; 112. Clearance notch;
20. Adjustment assembly; 21. Third end; 22. Fourth end; 23. Adjustment sleeve; 231. Second planar face; 232. Second boss; 24. Adjustment portion; 241. Fixing slot; 2411. First face; 2412. Second face; 242. First arc-shaped adjustment plate; 243. Connecting tube; 244. Second arc-shaped adjustment plate; 245. Cylindrical tube; 246. Limiting protrusion; 247. Annular baffle; 201. Sub-adjustment assembly;
30. Mounting assembly; 31. First annular boss; 40. Connecting assembly; 41. Fixing protrusion; 411. Third face; 412. Fourth face; 42. Torsion spring; 43. Pressing snap-fit member; 431. Pressing portion; 432. Second mounting hole; 44. Connecting shaft; 50. Guide groove; 60. Guide protrusion.

### DETAILED DESCRIPTION OF THE EMBODIMENTS

Only some exemplary embodiments will be briefly described below. As can be appreciated by those skilled in the art, the described embodiments can be modified in various ways without departing from the spirit or scope of the present application. Accordingly, the accompanying drawings and the description are considered illustrative in nature rather than limited.

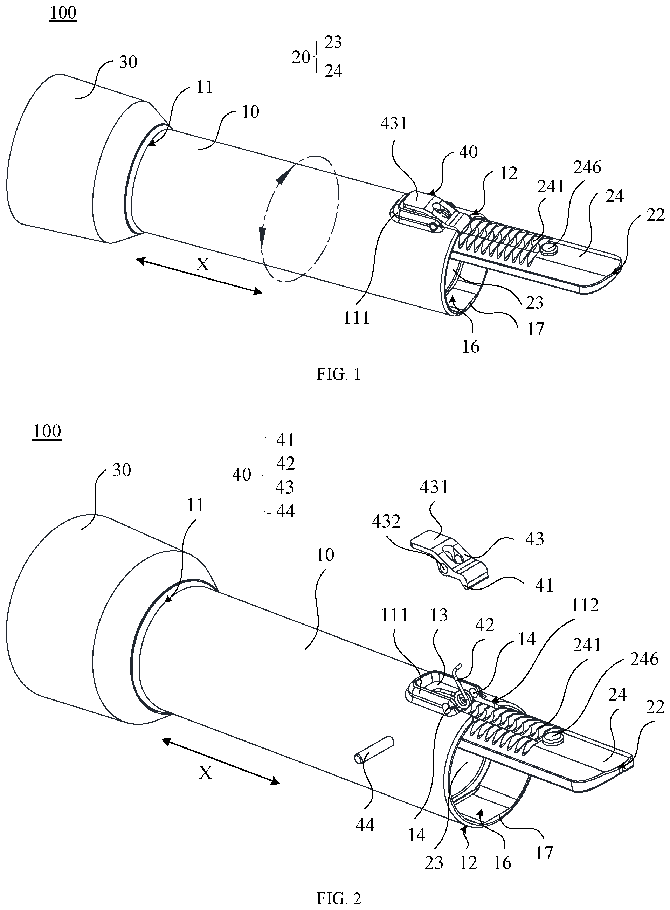

Embodiments of the present application provide a liquid flow sleeve. FIG. 1 shows a schematic structural diagram of a liquid flow sleeve according to an embodiment of the present application. FIG. 2 shows a schematic exploded view of the liquid flow sleeve according to an embodiment of the present application. Referring to FIGS. 1 and 2, a liquid flow sleeve 100 includes a sleeve body 10, an adjustment assembly 20, a mounting assembly 30, and a connecting assembly 40.

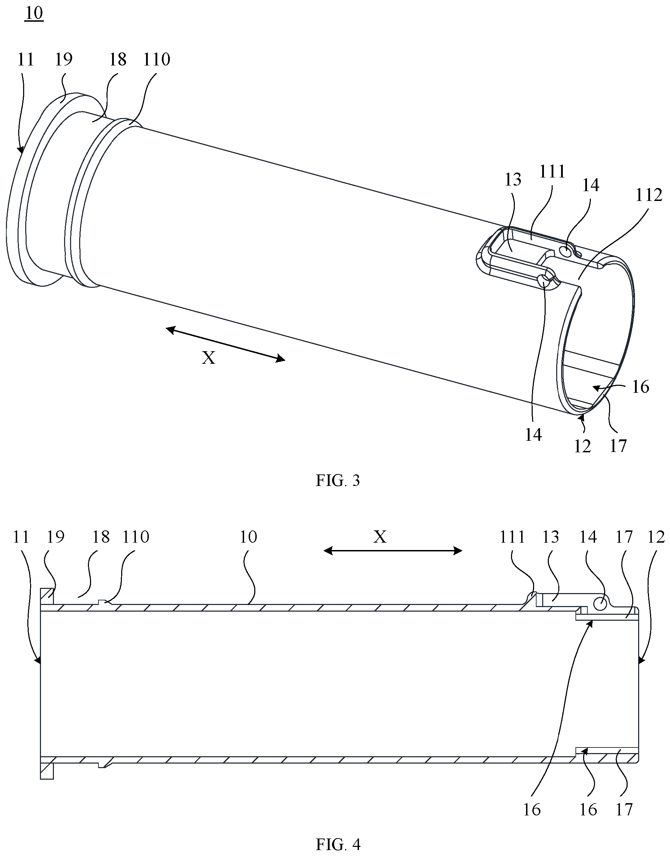

FIG. 3 shows a schematic structural diagram of a sleeve body according to an embodiment of the present application. FIG. 4 shows a schematic cross-sectional view of the sleeve body according to an embodiment of the present application. Referring to FIGS. 1 to 4, the sleeve body 10 extends in a first direction X, and the sleeve body 10 has a first end 11 and a second end 12 arranged opposite each other in the first direction X. The mounting assembly 30 is connected to the first end 11 of the sleeve body 10, and the mounting assembly 30 is configured to connect to the handle.

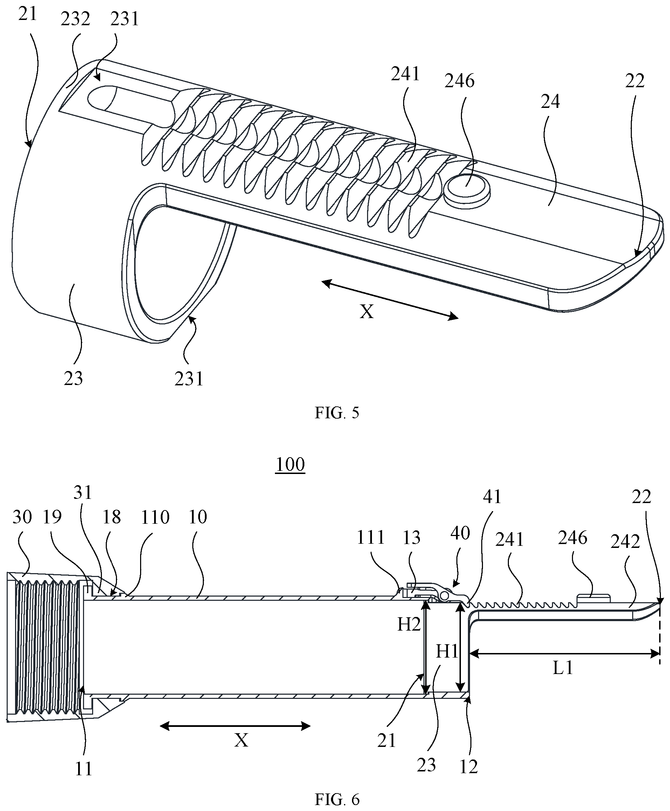

FIG. 5 shows a schematic structural diagram of an adjustment assembly according to an embodiment of the present application. Referring to FIG. 5, the adjustment assembly 20 has a third end 21 and a fourth end 22 arranged opposite each other in the first direction X. Referring to FIGS. 1 to 5, the connecting assembly 40 connects the sleeve body 10 and the adjustment assembly 20.

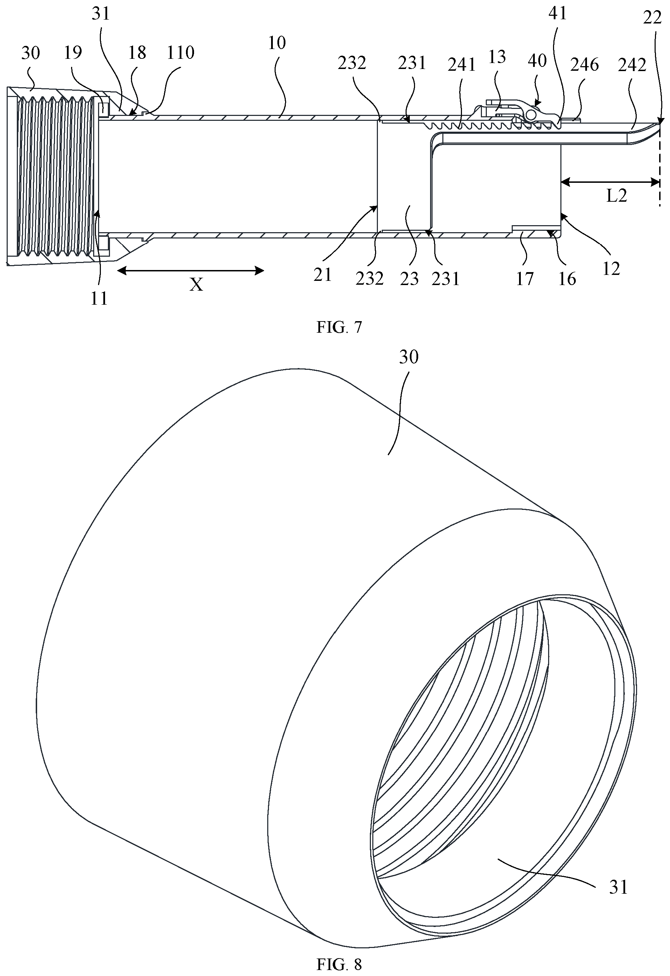

FIG. 6 shows a schematic cross-sectional view of the liquid flow sleeve according to an embodiment of the present application in one state. FIG. 7 shows a schematic cross-sectional view of the liquid flow sleeve according to an embodiment of the present application in another state. Referring to FIGS. 6 and 7, the adjustment assembly 20 is movable in the first direction X to change a maximum distance between an end portion of the second end 12 and an end portion of the fourth end 22 in the first direction X.

It should be noted that the maximum distance between the end portion of the second end 12 and the end portion of the fourth end 22 in the first direction X is the maximum distance between the end portion of the second end 12 and the end portion of the fourth end 22 in the first direction X when the position of the adjustment assembly 20 is fixed, that is, when the relative position of the sleeve body 10 and the adjustment assembly 20 is fixed. When the relative position of the sleeve body 10 and the adjustment assembly 20 changes, the maximum distance between the end portion of the second end 12 and the end portion of the fourth end 22 in the first direction X also changes. For example, in FIGS. 6 and 7, the relative position of the sleeve body 10 and the adjustment assembly 20 changes so that the maximum distance L1 between the end portion of the second end 12 and the end portion of the fourth end 22 in FIG. 6 is different from the maximum distance L2 between the end portion of the second end 12 and the end portion of the fourth end 22 in FIG. 7, where L1 is greater than L2.

In the embodiments of the present application, the sleeve body 10 is of a barrel-shaped structure, a receiving cavity of the sleeve body 10 extends in the first direction X, and both ends of the receiving cavity in the first direction X are open, so that a blade can pass through the receiving cavity of the sleeve body 10.

In an embodiment of the present application, the mounting assembly 30 is connected to the handle, so as to connect the liquid flow sleeve 100 to the handle. For example, the mounting assembly 30 and the handle may be connected in a threaded manner. For example, FIG. 8 shows a schematic structural diagram of a mounting assembly according to an embodiment of the present application. Referring to FIG. 8, the mounting assembly 30 has a barrel-shaped structure. The receiving cavity of the mounting assembly 30 extends in the first direction X. The inner side wall of the mounting assembly 30 has an internal thread, and one end of the handle has an external thread, so as to achieve a threaded connection between the mounting assembly 30 and the handle. Alternatively, the mounting assembly 30 has an external thread, and one end of the handle has an internal thread, so as to achieve a threaded connection between the mounting assembly 30 and the handle. In other implementations, the mounting assembly 30 and the handle can be connected in an interference-fit manner.

In the embodiments of the present application, the adjustment assembly 20 also has a receiving cavity with openings at both ends, so that the front end of the blade can extend out of the liquid flow sleeve, and the front end of the blade can be in contact with the tissue.

In the embodiments of the present application, since the sleeve body 10, the adjustment assembly 20 and the mounting assembly 30 all extend in the first direction X, the length of the liquid flow sleeve 100 is equal to the maximum distance between the end portion of the mounting assembly 30 away from the sleeve body 10 and the end portion of the fourth end 22 in the first direction X. The greater the maximum distance between the end portion of the second end 12 and the end portion of the fourth end 22 in the first direction X, the greater the length of the liquid flow sleeve 100.

In the embodiments of the present application, the connecting assembly 40 enables the third end 21 of the adjustment assembly 20 to be movable from the second end 12 of the sleeve body 10. The third end 21 of the adjustment assembly 20 is movably connected to the second end 12 of the sleeve body 10 in various forms. For example, the third end 21 of the adjustment assembly 20 has an external thread and the second end 12 of the sleeve body 10 has an internal thread, and the length of the threaded connection between the adjustment assembly 20 and the sleeve body 10 may be adjusted according to the required length of the liquid flow sleeve 100. Alternatively, the sleeve body 10 is sleeved on the adjustment assembly 20, the side wall of the sleeve body 10 has a plurality of through holes spaced in the first direction X, the side wall of the adjustment assembly 20 has a threaded hole, and when the length of the liquid flow sleeve needs to be adjusted, the threaded hole is adjusted to connect to different through holes, and then a bolt is controlled to pass through the through hole and then connect to the threaded hole, so as to achieve the adjustment of the length of the liquid flow sleeve.

In the embodiments of the present application, the length of the liquid flow sleeve 100 is positively correlated with the maximum distance between the end portion of the second end 12 and the end portion of the fourth end 22 in the first direction X. Once the liquid flow sleeve 100 and the blade are mounted, the length of the blade is fixed, and the length of the liquid flow sleeve 100 can be adjusted by controlling the adjustment assembly 20 to move in the first direction X. When the length of the liquid flow sleeve 100 is changed, the extension length of the blade can also be changed. The extension length of the blade can be adjusted by controlling the adjustment assembly 20 to move in the first direction X. There is no need to remove the liquid flow sleeve and replace it with a new liquid flow sleeve or blade. The liquid flow sleeve 100 is suitable for more scenarios, and the liquid flow sleeve 100 has a wider range of applications.

For example, by adjusting the overall length of the liquid flow sleeve 100, the extension length of the blade can be changed, thereby limiting the cutting depth and protecting soft tissue.

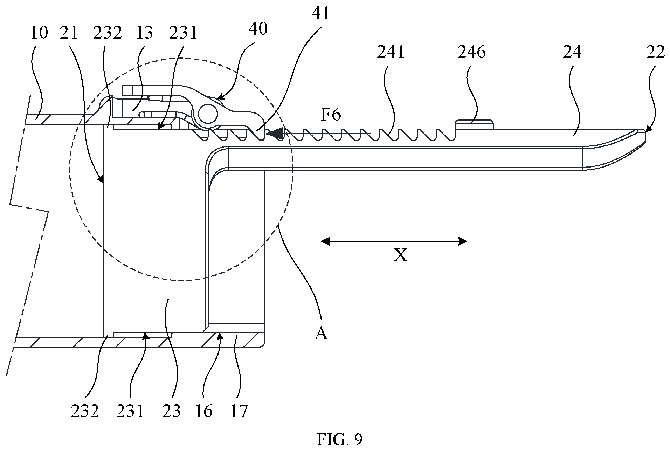

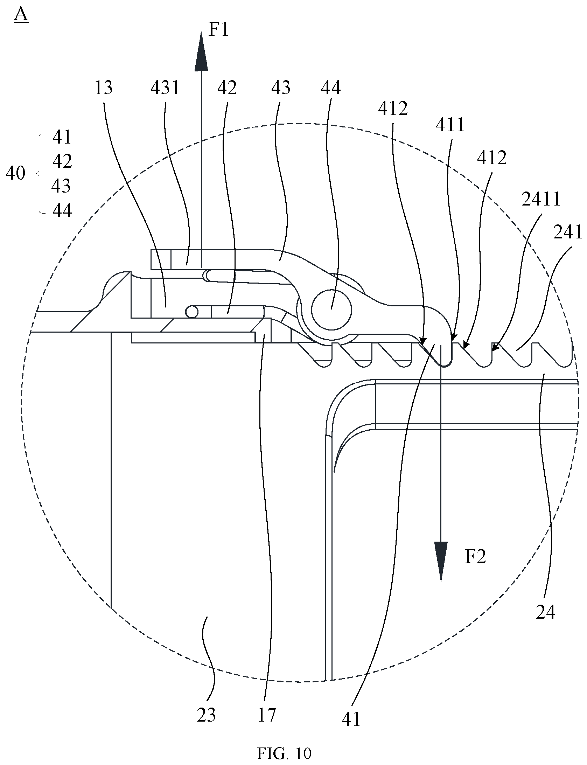

According to some embodiments of the present application, FIG. 9 shows a partial schematic cross-sectional view of the adjustment assembly fitting with the sleeve body according to an embodiment of the present application. FIG. 10 shows a partial enlarged view of part A in FIG. 9. Referring to FIGS. 1, 2, 5, 9 and 10, the adjustment assembly 20 includes an adjustment sleeve 23 and an adjustment portion 24. An outer side wall of the adjustment sleeve 23 fits against an inner side wall of the sleeve body 10, and the third end 21 is located in the adjustment sleeve 23. The adjustment portion 24 is connected to the other end of the adjustment sleeve 23 opposite the third end 21, the fourth end 22 is located at the other end of the adjustment portion 24 opposite the adjustment sleeve 23, and the outer side wall of the adjustment portion 24 has a plurality of fixing slots 241 spaced in the first direction X. One end of the connecting assembly 40 is connected to the outer side wall of the sleeve body 10, and the other end of the connecting assembly 40 has a fixing protrusion 41 movably located in a fixing slot 241. The fixing protrusion 41 fits with the fixing slots 241 such that the maximum distance between the end portion of the second end 12 and the end portion of the fourth end 22 in the first direction X is different when the fixing protrusion 41 is located in different fixing slots 241 among the plurality of fixing slots 241.

In the embodiments of the present application, the adjustment sleeve 23 and the adjustment portion 24 may be manufactured separately and then connected together, or the adjustment sleeve 23 and the adjustment portion 24 may be integrally formed.

For example, the adjustment portion 24 may be an arc-shaped plate extending in the first direction X, or the adjustment portion 24 may also be a sleeve extending in the first direction X.

In the embodiments of the present application, the outer side wall of the adjustment sleeve 23 fits against the inner side wall of the sleeve body 10, such that the adjustment sleeve 23 can move in the first direction X against the inner side wall of the sleeve body 10.

In the embodiments of the present application, since one end of the connecting assembly 40 is connected to the outer side wall of the sleeve body 10, the connecting assembly 40 is fixed when the fixing protrusion 41 is located in the fixing slot 241, so that the position of the adjustment assembly 20 and the sleeve body 10 is fixed, so as to achieve the connection between the sleeve body 10 and the adjustment assembly 20.

In the embodiments of the present application, when the length of the liquid flow sleeve needs to be adjusted, the fixing protrusion 41 is first controlled to disengage from the fixing slot 241, then the adjustment sleeve 23 is controlled to move in the first direction X against the inner side wall of the sleeve body 10 so that the relative position of the fixing slot 241 and the connecting assembly 40 changes, and when the length of the liquid flow sleeve is adjusted to reach a preset length, the fixing protrusion 41 is controlled to be located in the corresponding fixing slot 241, thereby adjusting the length of the liquid flow sleeve 100. If the adjustment sleeve 23 moves again, the fixing protrusion 41 will block the movement of the adjustment sleeve 23, thereby fixing the adjustment assembly 20.

According to some embodiments of the present application, referring to FIG. 3, the outer side wall of the sleeve body 10 has a fixing groove 13, and two opposite side walls of the fixing groove 13 each have a first mounting hole 14. Referring to FIGS. 2 and 10, the connecting assembly 40 includes a torsion spring 42, a pressing snap-fit member 43, and a connecting shaft 44. The torsion spring 42 is located in the fixing groove 13, and one of torsion arms of the torsion spring 42 abuts against a bottom surface of the fixing groove 13. The fixing protrusion 41 is connected to an end portion of the pressing snap-fit member 43, the other end of the pressing snap-fit member 43 opposite the fixing protrusion 41 has a pressing portion 431, the pressing snap-fit member 43 has a second mounting hole 432, and second mounting hole 432 is located between the fixing protrusion 41 and the pressing portion 431. The connecting shaft 44 sequentially passes through one first mounting hole 14 of the two first mounting holes 14, the second mounting hole 432, the center hole of the torsion spring 42, and the other first mounting hole 14 of the two first mounting holes 14. The other torsion arm of the torsion spring 42 abuts against a surface of the pressing portion 431 facing the fixing groove 13.

In the embodiments of the present application, the fixing groove 13 is located close to the second end 12 of the sleeve body 10 such that the connecting assembly 40 is closer to the adjustment portion 24, which facilitates the connection between the connecting assembly 40 and the adjustment portion 24.

In some embodiments of the present application, a portion of the outer side wall of the sleeve body 10 may be removed to form the fixing groove 13. In some other embodiments of the present application, a fourth annular boss 111 may be formed on the outer side wall of the sleeve body 10, and the fourth annular boss 111 and the outer side wall of the sleeve body 10 form the fixing groove 13.

In the embodiments of the present application, both torsion arms of the torsion spring 42 are located on the same side of the torsion spring 42, and both torsion arms of the torsion spring 42 can exert an elastic force on the components they contact.

In the embodiments of the present application, after the connecting assembly 40 is mounted, the pressing snap-fit member 43 can rotate around the connecting shaft 44. When the pressing snap-fit member 43 rotates, it can drive the fixing protrusion 41 to rotate, so that the fixing protrusion 41 can be disengaged from the fixing slot 241 or remain in the fixing slot 241.

In the embodiments of the present application, when in use, referring to FIG. 10, the torsion arms of the torsion spring 42 abut against the surface of the pressing portion 431 facing the fixing groove 13, the torsion arms of the torsion spring 42 exert an upward pushing force F1 on the pressing portion 431 to cause the pressing snap-fit member 43 to rotate around the connecting shaft 44, and the fixing protrusion 41 located at the other end of the pressing snap-fit member 43 moves towards the fixing slot 241 in the direction of F2 to cause the fixing protrusion 41 to be located in the fixing slot 241. If the adjustment sleeve 23 moves, the fixing protrusion 41 will block the movement of the adjustment sleeve 23, so as to fix the adjustment assembly 20.

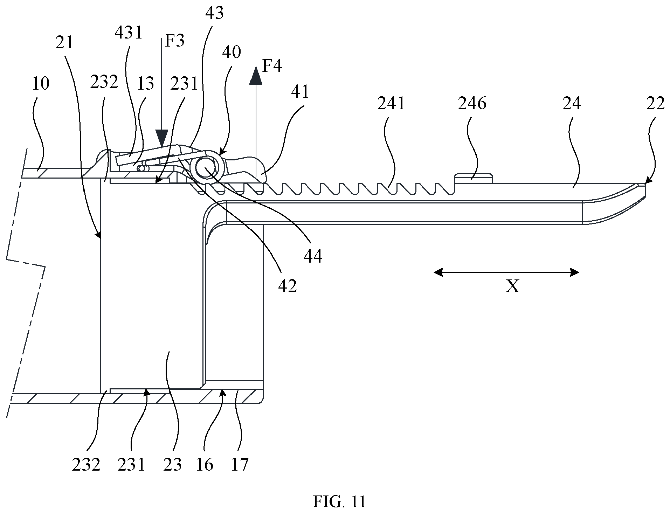

FIG. 11 shows a partial schematic cross-sectional view of the adjustment assembly fitting with the sleeve body in another state according to an embodiment of the present application. Referring to FIG. 11, when the length of the liquid flow sleeve 100 needs to be adjusted, the pressing snap-fit member 43 is pressed at the pressing portion 431, with a pressing force F3 exerted on the pressing portion 431 being greater than the pushing force F1 of the torsion arm of the torsion spring 42 on the pressing portion 431, so that the pressing portion 431 moves towards the bottom surface of the fixing groove 13 in a direction of F3, the pressing snap-fit member 43 rotates around the connecting shaft 44, the fixing protrusion 41 at the other end of the pressing snap-fit member 43 moves away from the fixing slot 241 in a direction of F4, so that the fixing protrusion 41 is disengaged from the fixing slot 241; and then the movement of the adjustment sleeve 23 is adjusted, and when the length of the adjusting liquid flow sleeve 100 reaches a preset length, the pressing of the pressing portion 431 is released, so that the pushing force F1 of the torsion arm of the torsion spring 42 on the pressing portion 431 pushes the pressing snap-fit member 43 to rotate again to enable the fixing protrusion 41 at the other end of the pressing snap-fit member 43 to move towards the fixing slot 241, so that the fixing protrusion 41 is located in the fixing slot 241.

In some embodiments of the present application, referring to FIG. 3, the outer side wall of the sleeve body 10 has a clearance notch 112. The clearance notch 112 is located at the second end 12 of the sleeve body 10, and the clearance notch 112 extends through one side wall of the fixing groove 13 and is in communication with the fixing groove 13. The clearance notch 112 provides clearance for the connecting assembly 40, allowing for more space for arrangement of the connecting assembly 40.

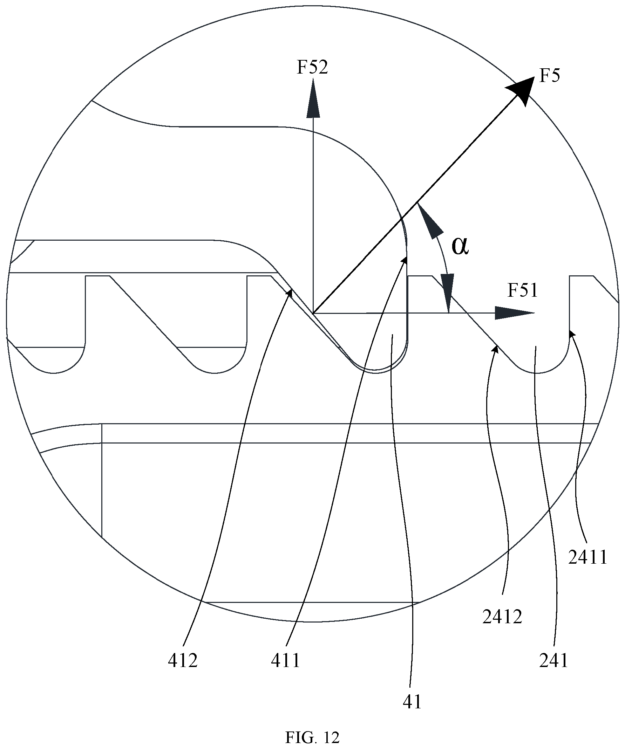

According to some embodiments of the present application, FIG. 12 shows a partial schematic cross-sectional view of a fixing protrusion fitting with a fixing slot according to an embodiment of the present application. Referring to FIGS. 10 and 12, the fixing slot 241 includes a first face 2411 and a second face 2412 facing each other, and an angle α between the first face 2411 and the second face 2412 is an acute angle. The fixing protrusion 41 includes a third face 411 and a fourth face 412 opposite each other. When the fixing protrusion 41 is located in the fixing slot 241, the third face 411 fits against the first face 2411, and the fourth face 412 fits against the second face 2412.

In some embodiments of the present application, the shape of the fixing slot 241 may be triangular as shown in the cross-sectional view of FIG. 10, in which case the first face 2411 is adjacent to and faces the second face 2412. In some other embodiments of the present application, the shape of the fixing slot 241 may be a rounded triangle as shown in the cross-sectional view of FIG. 10, that is, the first face 2411 is connected to the second face 2412 by an arc surface, in which case the first face 2411 faces the second face 2412. In some other embodiments of the present application, the shape of the fixing slot 241 may be trapezoidal as shown in the cross-sectional view of FIG. 10, that is, the first face 2411 is connected to the second face 2412 by a planar face, in which case the first face 2411 faces the second face 2412. In other embodiments of the present application, the shape of the fixing slot 241 may be rectangular as shown in the cross-sectional view of FIG. 10.

For example, the shape of the fixing protrusion 41 is the same as the shape of the fixing slot 241, which will not be repeated in the embodiments of the present application.

In some embodiments of the present application, the angle α between the first face 2411 and the second face 2412 is an acute angle. Under otherwise constant conditions, the fixing slot 241 is made small in shape with a small opening, such that the fixing protrusion 41 is less likely to disengage from the fixing slot 241. This ensures that the relative position of the adjustment assembly 20 and the sleeve body 10 is fixed during the surgical procedure, so as to avoid impact on the surgical procedure.

For example, the angle α between the first face 2411 and the second face 2412 is greater than or equal to 20° and less than or equal to 60°. For example, the angle α between the first face 2411 and the second face 2412 is 45°.

According to some embodiments of the present application, the first face 2411 is perpendicular to the first direction X. For the first face 2411 and the second face 2412 in the same fixing slot 241, the second face 2412 is closer to the third end 21, and the first face 2411 is closer to the fourth end 22.

In the embodiments of the present application, when the length of the liquid flow sleeve 100 needs to be increased without pressing the pressing portion 431, a force in the first direction X and away from the adjustment sleeve 23 may be exerted on the adjustment portion 24, and the second face 2412 of the fixing slot 241 may exert a pushing force F5 perpendicular to the second face 2412 on the fixing protrusion 41. F5 may be decomposed into a first component F51 parallel to the first direction X and a second component F52 perpendicular to the first direction X, and when the pushing force F5 is large enough, the second component F52 may push the fixing protrusion 41 to move in a direction of the second component F52 away from the fixing slot 241, so that the fixing protrusion 41 is disengaged from the fixing slot 241, thereby increasing the length of the liquid flow sleeve 100.

During the surgical procedure, when the tissue at the surgical site exerts a force in the first direction X and towards the adjustment sleeve 23 on the adjustment portion 24, the first face 2411 of the fixing slot 241 may exert a pushing force F6 perpendicular to the first face 2411 on the fixing protrusion 41. The pushing force F6 has no component perpendicular to the first direction X, making it impossible for the fixing protrusion 41 to disengage from the fixing slot 241. Meanwhile, during the surgical procedure, the tissue at the surgical site will hardly exert a force on the adjustment portion 24 in the first direction X and away from the adjustment sleeve 23. This ensures that the relative position of the adjustment assembly 20 and the sleeve body 10 is fixed during the surgical procedure, so as to avoid impact on the surgical procedure.

According to some other embodiments of the present application, the angles of the first face 2411 and the second face 2412 with the first direction X are both greater than 30 degrees.

In the embodiments of the present application, the angles of the first face 2411 and the second face 2412 with the first direction X are both greater than 30 degrees. As mentioned above, the length of the liquid flow sleeve 100 may be increased or decreased without pressing the pressing portion 431. Meanwhile, since the angles of the first face 2411 and the second face 2412 with the first direction X are both greater than 30 degrees, the component perpendicular to the first direction X is smaller during the adjustment process so that a larger force is required to adjust the length of the liquid flow sleeve 100. During the surgical procedure, and the force exerted by the tissue at the surgical site on the adjustment portion 24 is generally small and insufficient to push the adjustment assembly 20 to move. This ensures that the relative position of the adjustment assembly 20 and the sleeve body 10 is fixed during the surgical procedure, so as to avoid impact on the surgical procedure.

For example, the angle between the first face 2411 and the first direction X and the angle between the second face 2412 and the first direction X may be equal or unequal. For example, the angles of the first face 2411 and the second face 2412 with the first direction X is 75 degrees; or the angle between the first face 2411 and the first direction X is 80 degrees, and the angle between the second face 2412 and the first direction X is 60 degrees.

In some embodiments of the present application, referring to FIGS. 1, 2, 4, 10 and 11, the extension direction of the connecting shaft 44 is perpendicular to the first direction X.

In the embodiments of the application, since the extension direction of the connecting shaft 44 is perpendicular to the first direction X, the fixing protrusion 41 at the end portion of the pressing snap-fit member 43 can extend out of the second end 12, making it easier for the fixing protrusion 41 to fit with the fixing slot 241, which is more convenient.

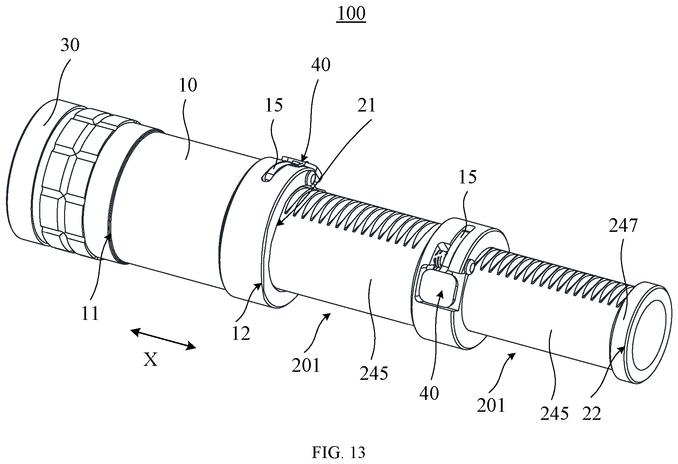

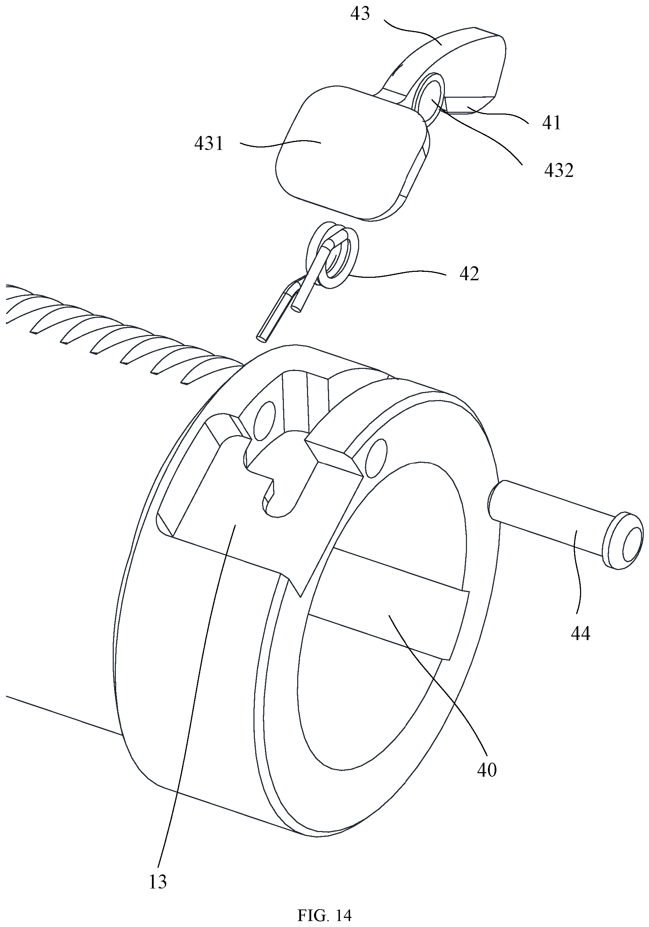

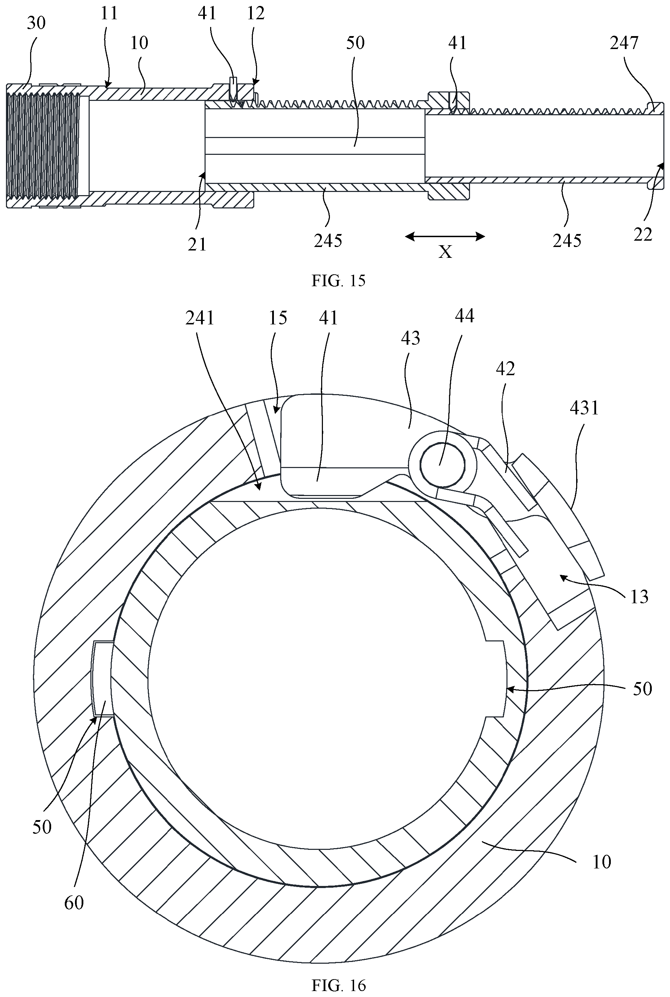

In some other embodiments of the present application, FIG. 13 shows a schematic structural diagram of a further liquid flow sleeve according to an embodiment of the present application. FIG. 14 shows a partial schematic exploded view of the further liquid flow sleeve according to an embodiment of the present application. FIG. 15 shows a schematic cross-sectional view of the further liquid flow sleeve according to an embodiment of the present application. Referring to FIGS. 13 to 15, the extension direction of the connecting shaft 44 is parallel to the first direction X, and the side wall of the sleeve body 10 has a fixing through hole 15 for the fixing protrusion 41 to extend into the inner cavity of the sleeve body 10.

In the embodiments of the present application, the extension direction of the connecting shaft 44 is parallel to the first direction X, making the direction of length of the connecting assembly 40 perpendicular to the first direction X. Meanwhile, the fixing protrusion 41 may fit with the fixing slot 241 through the fixing through hole 15, allowing the part of the fixing protrusion 41 fitting with the fixing slot 241 to be located inside the sleeve body 10 and not exposed to the outside. This is aesthetically pleasing and also prevents the part of the fixing protrusion 41 fitting with the fixing slot 241 from being bumped and thus affecting the safety of the surgical procedure.

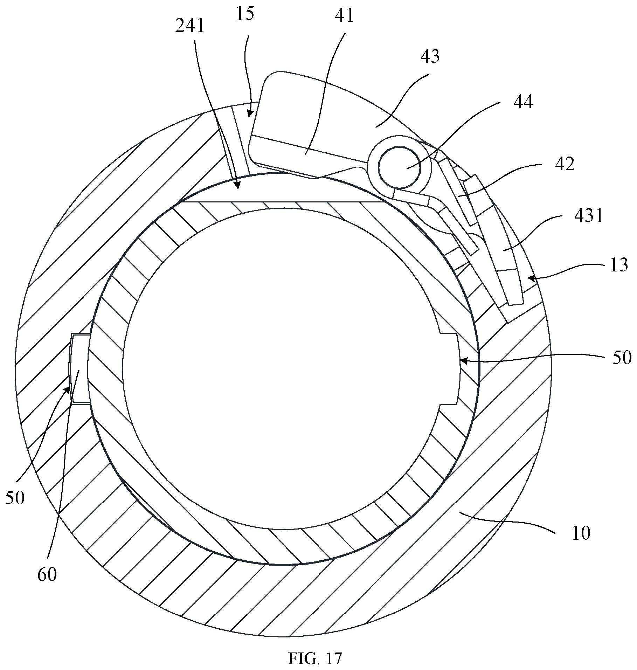

FIG. 16 shows a schematic cross-sectional view of the further liquid flow sleeve according to an embodiment of the present application in another cross section. FIG. 17 shows a schematic cross-sectional view of the further liquid flow sleeve according to an embodiment of the present application in another state. Referring to FIGS. 16 and 17, when the pressing portion 431 is not pressed, the fixing protrusion 41 is located in the fixing slot 241, so that the adjustment assembly 20 cannot move in the first direction X, thereby ensuring that the relative position of the adjustment assembly 20 and the sleeve body 10 is fixed during the surgical procedure. After the pressing portion 431 is pressed, the fixing protrusion 41 moves in the fixing through hole 15 and disengages from the fixing slot 241, thereby allowing the adjustment assembly 20 to move in the first direction X to adjust the length of the liquid flow sleeve 100.

According to some embodiments of the present application, referring to FIGS. 1, 2 and 5 to 7, the adjustment portion 24 includes a first arc-shaped adjustment plate 242. The first arc-shaped adjustment plate 242 extends in the first direction X, one end of the first arc-shaped adjustment plate 242 in the first direction X is connected to the adjustment sleeve 23, and the other end of the first arc-shaped adjustment plate 242 in the first direction X is a free end.

In the embodiments of the present application, the adjustment portion 24 does not completely cover the blade in the circumferential direction, which can provide an operator with a better view during the surgical procedure to facilitate the surgical procedure.

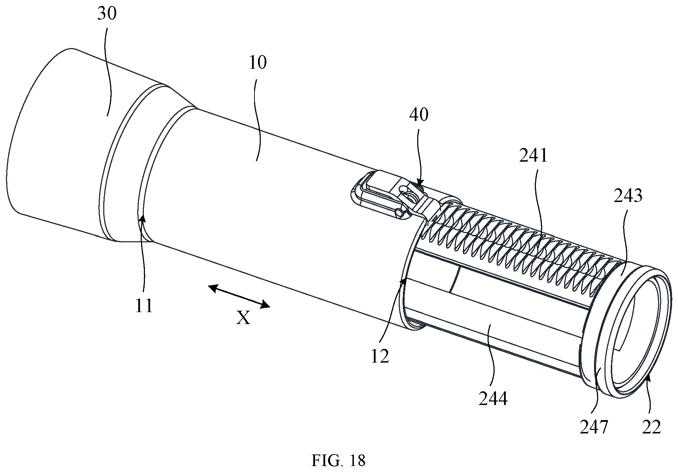

According to some other embodiments of the present application, FIG. 18 shows a schematic structural diagram of the further liquid flow sleeve according to an embodiment of the present application. Referring to FIG. 18, the adjustment portion 24 includes a connecting tube 243 and a plurality of second arc-shaped adjustment plates 244. The plurality of second arc-shaped adjustment plates 244 extend in the first direction X, one end of each of the plurality of second arc-shaped adjustment plates 244 in the first direction X is connected to the adjustment sleeve 23, the other end of each of the plurality of second arc-shaped adjustment plates 244 in the first direction X is connected to the connecting tube 243, the plurality of second arc-shaped adjustment plates 244 are spaced along the circumference of the adjustment sleeve 23, and the fixing slots 241 are located in at least one second arc-shaped adjustment plate 244 of the plurality of second arc-shaped adjustment plates 244.

In the embodiments of the present application, the adjustment portion 24 is designed with a hollowed-out structure by means of the plurality of second arc-shaped adjustment plates 244 and connecting tubes 243. During the surgical procedure, the operator can observe from the hollowed-out part, which can give the operator a certain view, and also improve the strength of the adjustment portion 24.

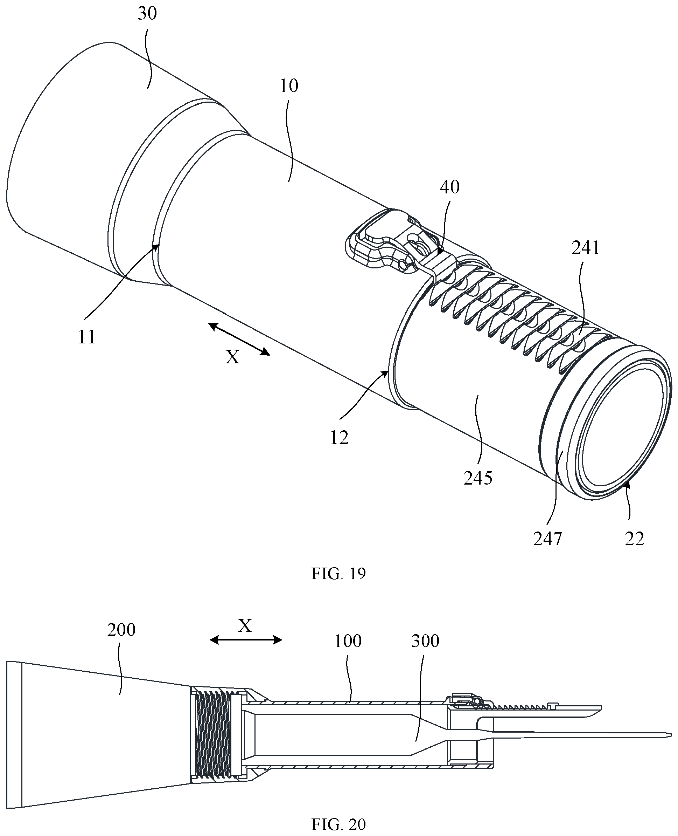

According to some other embodiments of the present application, FIG. 19 shows a schematic structural diagram of the further liquid flow sleeve according to an embodiment of the present application. Referring to FIGS. 13 and 19, the adjustment portion 24 includes a cylindrical tube 245. The cylindrical tube 245 extends in the first direction X.

In the embodiments of the present application, the adjustment portion 24 is directly configured as the cylindrical tube 245, which has a simple structure and is easier to manufacture, and the adjustment portion 24 has higher strength and is not easily damaged.

According to some embodiments of the present application, referring to FIG. 5, the outer side wall of the adjustment portion 24 has a limiting protrusion 246, the limiting protrusion 246 and a plurality of fixing slots 241 are arranged in the first direction X, and the limiting protrusion 246 is located on a side of the plurality of fixing slots 241 close to the fourth end 22.

In the embodiments of the present application, during the movement of the adjustment assembly 20 towards the sleeve body 10 in the first direction X, the distance between the limiting protrusion 246 and the second end 12 of the sleeve body 10 gets closer and closer, and when the second end 12 abuts against the limiting protrusion 246, the adjustment assembly 20 cannot move towards the sleeve body 10, so as to prevent the adjustment assembly 20 from completely entering the inner cavity of the sleeve body 10 and thus making it impossible to remove the adjustment assembly 20.

In the embodiments of the present application, referring to FIG. 18, when the adjustment portion 24 includes a connecting tube 243 and a plurality of second arc-shaped adjustment plates 244, an annular baffle 247 may be provided at an end of the connecting tube 243 away from the sleeve body 10. The annular baffle 247 can also prevent the adjustment assembly 20 from completely entering the inner cavity of the sleeve body 10 and thus making it impossible to remove the adjustment assembly 20.

In the embodiments of the present application, when the sleeve body 10 and the adjustment assembly 20 are mounted, with the annular baffle 247 being not yet mounted on the connecting tube 243, the adjustment sleeve 23 is first inserted into the interior of the sleeve body 10 from the first end 11 of the sleeve body 10, and after the adjustment sleeve 23 extends out from the second end 12 of the sleeve body 10, the annular baffle 247 is then mounted on the connecting tube 243.

For example, the connecting tube 243 may be connected to the annular baffle 247 by means of welding; or the connecting tube 243 may be connected to the annular baffle 247 by means of adhesive bonding.

In the embodiments of the present application, referring to FIGS. 13 and 19, when the adjustment portion 24 includes a cylindrical tube 245, an annular baffle 247 may also be provided at an end of the cylindrical tube 245 away from the sleeve body 10, so as to prevent the adjustment assembly 20 from being completely inserted into the inner cavity of the sleeve body 10 and thus making it impossible to remove the adjustment assembly 20.

In the embodiments of the present application, when the sleeve body 10 and the adjustment assembly 20 are mounted, with the annular baffle 247 being not yet connected to the cylindrical tube 245, an end of the cylindrical tube 245 away from the adjustment sleeve 23 is first inserted into the interior of the sleeve body 10 from the first end 11 of the sleeve body 10, and after the end of the cylindrical tube 245 away from the adjustment sleeve 23 extends out from the second end 12 of the sleeve body 10, the annular baffle 247 is then connected to the cylindrical tube 245.

For example, the annular baffle 247 may be connected to the cylindrical tube 245 by means of welding; or the annular baffle 247 may be connected to the cylindrical tube 245 in a threaded manner; or the annular baffle 247 may be interference-fitted with the cylindrical tube 245.

Referring to FIGS. 1 to 5, the inner side wall of the sleeve body 10 has a first planar face 16, the first planar face 16 is parallel to the first direction X, and the first planar face 16 extends to the end face of the second end 12; and the outer side wall of the adjustment sleeve 23 has a second planar face 231, the second planar face 231 extends to the end face of the adjustment sleeve 23 opposite the third end 21, and the first planar face 16 fits against the second planar face 231.

During the surgical procedure, it is essential to ensure that the sleeve body 10 and the adjustment assembly 20 cannot rotate relative to each other, so as to avoid the mutual rotation between the sleeve body 10 and the adjustment assembly 20 affecting the operator's line of sight and causing adverse effects on the surgical procedure. In the embodiments of the present application, the inner side wall of the sleeve body 10 has a first planar face 16, the outer side wall of the adjustment sleeve 23 has a second planar face 231, and the first planar face 16 fits against the second planar face 231, so that when the sleeve body 10 and the adjustment assembly 20 need to rotate relative to each other, since the other parts of the inner side wall of the sleeve body 10 and the other parts of the outer side wall of the adjustment sleeve 23 are all arc surfaces, the first planar face 16 and the second planar face 231 will block the related rotation between the sleeve body 10 and the adjustment assembly 20. This ensures that the relative position between the sleeve body 10 and the adjustment assembly 20 is fixed, so as to avoid impact on the surgical procedure.

For example, the inner side wall of the sleeve body 10 may have a first planar face 16; or the inner side wall of the sleeve body 10 may have a plurality of first planar faces 16, and the plurality of first planar faces 16 are evenly spaced along the circumference of the sleeve body 10.

Referring to FIGS. 1 to 7, the inner side wall of the sleeve body 10 has a first boss 17 protruding into the inner cavity of the sleeve body 10, and a surface of the first boss 17 facing the inner cavity of the sleeve body 10 is the first planar face 16. In a direction perpendicular to the second planar face 231, a height H1 of the other end of the adjustment sleeve 23 opposite the third end 21 is less than a height H2 of the third end 21, such that the third end 21 of the adjustment sleeve 23 forms a second boss 232. The second boss 232 is located on a side of the first boss 17 away from the second end 12. An orthographic projection of the first boss 17 on the first surface at least partially overlaps with the orthographic projection of the second boss 232 on the first surface. The first surface is perpendicular to the first direction X.

In the embodiments of the present application, the orthographic projection of the first boss 17 on the first surface represents a projection of the first boss 17 on the first planar face when the projection lines are parallel to each other and perpendicular to the first planar face. Similarly, the orthographic projection of the second boss 232 on the first surface represents a projection of the second boss 232 on the first planar face when the projection lines are parallel to each other and perpendicular to the first planar face.

In the embodiments of the present application, a first boss 17 is provided in the inner cavity of the sleeve body 10, a first planar face 16 is formed by the first boss 17, and a second boss 232 is provided at the third end 21 of the adjustment sleeve 23. During the movement of the adjustment sleeve 23 towards the adjustment portion 24 in the first direction X, the distance between the first boss 17 and the second boss 232 gets closer and closer. Since the orthographic projection of the first boss 17 on the first surface at least partially overlaps with the orthographic projection of the second boss 232 on the first surface, when the first boss 17 and the second boss 232 finally abut against each other, the adjustment assembly 20 cannot move, so as to prevent the adjustment assembly 20 from completely disengaging from the inner cavity of the sleeve body 10 and thus affecting the integrity of the liquid flow sleeve 100.

In the embodiments of the present application, when the sleeve body 10 and the adjustment assembly 20 are mounted, with the limiting protrusion 246 being not yet mounted on the outer side wall of the adjustment portion 24, the fourth end 22 of the adjustment assembly 20 is first inserted into the interior of the sleeve body 10 from the first end 11 of the sleeve body 10, and after the fourth end 22 of the adjustment assembly 20 extends out from the second end 12 of the sleeve body 10, the limiting protrusion 246 is then mounted at the corresponding position on the outer side wall of the adjustment portion 24.

For example, the limiting protrusion 246 may be welded to the outer side wall of the adjustment portion 24; or the limiting protrusion 246 may be connected to the outer side wall of the adjustment portion 24 in a threaded manner. For example, the limiting protrusion 246 may be a bolt, and the outer side wall of the adjustment portion 24 has a corresponding threaded hole, so that the limiting protrusion 246 may be connected to the outer side wall of the adjustment portion 24 in a threaded manner; or the limiting protrusion 246 may be bonded to the outer side wall of the adjustment portion 24.

In some embodiments of the present application, the sleeve body 10 and the mounting assembly 30 may be manufactured separately and then connected together. For example, referring to FIGS. 3, 4, 6 and 7, the mounting assembly 30 is sleeved on the first end 11 of the sleeve body 10, and the outer side wall of the sleeve body 10 has a circle of mounting groove 18 around the first end 11. The mounting groove 18 surrounds the sleeve body 10 along the circumference of the sleeve body 10. Referring to FIG. 8, a circle of first annular boss 31 is provided on an inner side wall of an end of the mounting assembly 30 facing the sleeve body 10. The first annular boss 31 is located in the mounting groove 18.

In the embodiments of the present application, after the sleeve body 10 and the mounting assembly 30 are mounted, the first annular boss 31 is located in the mounting groove 18, so that the sleeve body 10 and the mounting assembly 30 cannot move in the first direction X, so as to avoid the change in the extension length of a shank affecting the surgical procedure. However, the sleeve body 10 and the mounting assembly 30 can rotate relative to each other, so that after the mounting assembly 30 is connected to the handle 200, the sleeve body 10 can be rotated to adjust the view of the operator.

In some embodiments of the present application, when the first annular boss 31 is located in the mounting groove 18, the sleeve body 10 and the mounting assembly 30 may be interference-fitted, so that the sleeve body 10 and the mounting assembly 30 cannot be easily rotated. When a large external force is exerted, the sleeve body 10 and the mounting assembly 30 can be rotated, so as to prevent the sleeve body 10 and the mounting assembly 30 from easily rotating during the surgical procedure and thus affecting the surgical procedure.

In some other embodiments of the present application, a rubber ring or a silicone ring may be provided between the first annular boss 31 and the mounting groove 18, which also makes it difficult for the sleeve body 10 and the mounting assembly 30 to rotate easily, so that the sleeve body 10 and the mounting assembly 30 can rotate relative to each other when a large external force is exerted.

Referring to FIGS. 3 and 4, the outer side wall of the sleeve body 10 has a second annular boss 19 and a third annular boss 110 spaced in the first direction X, and a mounting groove 18 is formed between the second annular boss 19 and the third annular boss 110.

In the embodiments of the present application, providing a second annular boss 19 and a third annular boss 110 on the outer side wall of the sleeve body 10 makes it easier to form the mounting groove 18, and also makes it easier to mount the sleeve body 10 and the mounting assembly 30.

For example, the third annular boss 110 is first welded to the outer side wall of the sleeve body 10, then the first end 11 of the sleeve body 10 is inserted into the mounting assembly 30 from an end of the mounting assembly 30 having the first annular boss 31, and then the second annular boss 19 is mounted on the outer side wall of the sleeve body 10 from an end of the mounting assembly 30 away from the first annular boss 31, thereby achieving the mounting of the sleeve body 10 and the mounting assembly 30.

For example, the second annular boss 19 may be connected to the outer side wall of the sleeve body 10 by means of welding; or the second annular boss 19 may be connected to the outer side wall of the sleeve body 10 in a threaded manner; or the second annular boss 19 may be interference-fitted with the outer side wall of the sleeve body 10.

For example, the third annular boss 110 and the outer side wall of the sleeve body 10 may be integrally formed.

Referring to FIG. 13, patterns may be formed on the outer side wall of the mounting assembly 30, so that the operator can operate the liquid flow sleeve 100 stably without slipping.

In some other embodiments of the present application, the sleeve body 10 and the mounting assembly 30 may be integrally formed.

Referring to FIGS. 16 and 17, one of the inner side wall of the sleeve body 10 and the outer side wall of the adjustment sleeve 23 has a guide groove 50, and the guide groove 50 extends in the first direction X; and the other of the inner side wall of the sleeve body 10 and the outer side wall of the adjustment sleeve 23 has a guide protrusion 60, and the guide protrusion 60 is movably located within the guide groove 50.

For example, the inner side wall of the sleeve body 10 has a guide groove 50, and the outer side wall of the adjustment sleeve 23 has a guide protrusion 60; or the outer side wall of the adjustment sleeve 23 has a guide groove 50, and the inner side wall of the sleeve body 10 has a guide protrusion 60.

In the embodiments of the present application, the guide protrusion 60 is movably located within the guide groove 50, so that the inner side wall of the sleeve body 10 and the adjustment sleeve 23 cannot rotate relative to each other. This ensures that the relative position between the sleeve body 10 and the adjustment assembly 20 is fixed, so as to avoid impact on the surgical procedure. Meanwhile, the guide groove 50 extends in the first direction X, so that the sleeve body 10 and the adjustment assembly 20 can move relative to each other in the first direction X, so as to adjust the length of the liquid flow sleeve 100.

In the embodiments of the present application, the guide protrusion 60 may be in an elongated shape extending in the first direction X, or the guide protrusion 60 may be a cylindrical or square protrusion.

Referring to FIG. 13, the adjustment assembly 20 includes a plurality of sub-adjustment assemblies 201. The sub-adjustment assemblies 201 are connected by a connecting assembly 40, and any two adjacent sub-adjustment assemblies 201 are movably connected in the first direction X.

In the embodiments of the present application, a plurality of sub-adjustment assemblies 201 are arranged, and any two adjacent sub-adjustment assemblies 201 are movably connected in the first direction X. This allows the adjustment assembly 20 to have a wider range of adjustable lengths. The more adjustable lengths the liquid flow sleeve 100 has, the more lengths the shank can extend out of the liquid flow sleeve 100, so that the liquid flow sleeve 100 has a wider range of applications.

For example, the same mounting groove as in the sleeve body 10 may be provided in the outer side wall of the sub-adjustment assembly 201 so that the connecting assembly 40 can connect two adjacent sub-adjustment assemblies 20.

Referring to FIGS. 16 and 17, a guide groove 50 may be arranged in the inner side wall of the adjustment sleeve 23 of one sub-adjustment assembly 201 of the two adjacent sub-adjustment assemblies 201, and a guide protrusion 60 may be arranged on the outer side wall of the adjustment sleeve 23 of the other sub-adjustment assembly 201, so that the two adjacent sub-adjustment assemblies 201 cannot rotate relative to each other, thus avoiding impact on the surgical procedure.

An embodiment of the present application provides a medical ultrasonic scalpel. FIG. 20 shows a schematic structural diagram of the medical ultrasonic scalpel according to the embodiment of the present application. Referring to FIG. 20, the medical ultrasonic scalpel includes a liquid flow sleeve 100, a handle 200, and a blade 300. The handle 200 is connected to the mounting assembly 30 of the liquid flow sleeve 100, the blade 300 is connected to the handle 200, and the liquid flow sleeve 100 is sleeved on the blade 300.

In some embodiments of the present application, an end of the handle 200 connected to the liquid flow sleeve 100 has a mounting groove or mounting hole, and the central axis of the mounting groove or mounting hole is coaxial with the central axis of the handle 200. The opening of the mounting groove or mounting hole runs through an end of the handle 200 connected to the liquid flow sleeve 100. An outer side wall of the mounting groove or mounting hole has an external thread, and an inner side wall of the mounting groove or mounting hole has an internal thread. An outer side wall of the blade 300 has an external thread, and an inner side wall of the liquid flow sleeve 100 has an internal thread. The external thread of the blade 300 is connected to the internal thread of the handle 200 in a threaded manner, and the internal thread of the liquid flow sleeve 100 is connected to the external thread of the handle 200 in a threaded manner.

In some other embodiments of the present application, the liquid flow sleeve 100 may be connected to the handle 200 in an interference-fit manner, and the blade 300 may be connected to the handle 200 in a threaded manner described above; or the liquid flow sleeve 100 may be connected to the handle 200 in a threaded manner described above, and the blade 300 may be connected to the handle 200 in an interference-fit manner; or the connection between the liquid flow sleeve 100 and the handle 200 and the connection between the blade 300 and the handle 200 may be achieved in an interference-fit manner.

In the medical ultrasonic scalpel according to the embodiments of the present application, the length of the liquid flow sleeve 100 can be adjusted to adjust the length of the exposed portion of the blade 300, so as to adjust the cutting depth of the medical ultrasonic scalpel, so that the medical ultrasonic scalpel can achieve cutting at different depths and has a wider range of applications.

An embodiment of the present application provides a robot-assisted ultrasonic scalpel system. The robot-assisted ultrasonic scalpel system includes a robot-assisted surgical device, and the medical ultrasonic scalpel described in the above embodiments. The robot-assisted surgical device is connected to the blade of the medical ultrasonic scalpel to control the movement of the blade.

It should be understood that, in this description, the orientations or positional relationships or dimensions denoted by the terms, such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial" and "circumferential", are the orientations or positional relationships or dimensions shown on the basis of the drawings, and these terms are used merely for ease of description, rather than indicating or implying that the device or element referred to must have particular orientations and be constructed and operated in the particular orientations, and therefore should not be construed as limiting the scope of protection of the present application.

In addition, the terms such as "first", "second" and "third" are merely for descriptive purposes and should not be construed as indicating or implying relative importance or implicitly indicating the number of technical features indicated. Thus, the features defined with "first", "second" and "third" may explicitly or implicitly include one or more features. In the description of the present application, the term "plurality of" means two or more, unless expressly and specifically limited otherwise.

In the present application, unless expressly stated or limited otherwise, the terms such as "mounting", "connection", "connected" and "fixing" should be interpreted broadly, for example, either a fixed or detachable connection, or integration; may be a mechanical connection, or an electrical connection, or communication; and may be a direct connection or an indirect connection by means of an intermediate medium, or may be internal communication between two elements or interaction between the two elements. For those of ordinary skills in the art, the specific meaning of the terms mentioned above in the present application may be construed according to specific circumstances.

In the present application, unless expressly stated or limited otherwise, the expression of the first feature being "above" or "below" the second feature may include the case where the first feature is in direct contact with the second feature, and may also include the case where the first and second features are not in direct contact but are contacted via another feature therebetween. Furthermore, the first feature being "over", "above" or "on" the second feature includes the case where the first feature is directly or obliquely above the second feature, or merely indicates that the first feature is at a higher level than the second feature. The first feature being "below", "under" or "beneath" the second feature includes the case where the first feature is directly or obliquely below the second feature, or merely indicates that the first feature is at a lower level than the second feature.

This description provides many different embodiments or examples that can be used to implement the present application. It should be understood that these various embodiments or examples are purely illustrative and are not intended to limit the scope of protection of the present application in any way. On the basis of the disclosure of the description of the present application, those skilled in the art will be able to conceive of various changes or substitutions. Any changes or substitutions shall fall within the scope of protection of the present application. Therefore, the scope of protection of the present application shall be subject to the scope of protection of the claims.

## Claims

1. A liquid flow sleeve, the liquid flow sleeve (100) comprising:
a sleeve body (10) extending in a first direction X, the sleeve body (10) having a first end (11) and a second end (12) arranged opposite each other in the first direction X;
an adjustment assembly (20) having a third end (21) and a fourth end (22) arranged opposite each other in the first direction X;
a mounting assembly (30) connected to the first end (11) of the sleeve body (10), the mounting assembly (30) being configured to connect to a handle; and
a connecting assembly (40) connecting the sleeve body (10) and the adjustment assembly (20);
wherein the adjustment assembly (20) is movable in the first direction X to change a maximum distance between an end portion of the second end (12) and an end portion of the fourth end (22) in the first direction X.

2. The liquid flow sleeve according to claim 1, wherein the adjustment assembly (20) comprises:
an adjustment sleeve (23), an outer side wall of the adjustment sleeve (23) fitting against an inner side wall of the sleeve body (10), and the third end (21) being located at the adjustment sleeve (23); and
an adjustment portion (24) connected to the adjustment sleeve (23), the adjustment portion (24) and the third end (21) being located at two opposite ends of the adjustment sleeve (23) in the first direction X, the fourth end (22) being located at an opposite end from the connection of the adjustment portion (24) with the adjustment sleeve (23), and an outer side wall of the adjustment portion (24) having a plurality of fixing slots (241) spaced in the first direction X; and
one end of the connecting assembly (40) is connected to an outer side wall of the sleeve body (10), and the other end of the connecting assembly (40) has a fixing protrusion (41) movably located in the fixing slot (241), wherein the fixing protrusion (41) fits with the fixing slots (241) such that the maximum distance between the end portion of the second end (12) and the end portion of the fourth end (22) in the first direction X is different when the fixing protrusion (41) is located in different fixing slots (241) among the plurality of fixing slots (241).

3. The liquid flow sleeve according to claim 2, wherein the outer side wall of the sleeve body (10) has a fixing groove (13), two opposite side walls of the fixing groove (13) each having a first mounting hole (14); and the connecting assembly (40) comprises:
a torsion spring (42) located in the fixing groove (13), one of torsion arms of the torsion spring (42) abutting against a bottom surface of the fixing groove (13);
a pressing snap-fit member (43), the fixing protrusion (41) being connected to an end portion of the pressing snap-fit member (43), the other end of the pressing snap-fit member (43) opposite the fixing protrusion (41) having a pressing portion (431), the pressing snap-fit member (43) having a second mounting hole (432), and the second mounting hole (432) being located between the fixing protrusion (41) and the pressing portion (431); and
a connecting shaft (44) sequentially passing through one first mounting hole (14) of the two first mounting holes (14), the second mounting hole (432), a center hole of the torsion spring (42), and the other first mounting hole (14) of the two first mounting holes (14);
wherein the other torsion arm of the torsion spring (42) abuts against a surface of the pressing portion (431) facing the fixing groove (13).

4. The liquid flow sleeve according to claim 3, wherein each of the fixing slots (241) comprises a first face (2411) and a second face (2412) facing each other, and an angle between the first face (2411) and the second face (2412) is an acute angle; and
the fixing protrusion (41) comprises a third face (411) and a fourth face (412) opposite each other, and when the fixing protrusion (41) is located in the fixing slot (241), the third face (411) fits against the first face (2411), and the fourth face (412) fits against the second face (2412).

5. The liquid flow sleeve according to claim 4, wherein the first face (2411) is perpendicular to the first direction X, and for the first face (2411) and the second face (2412) in the same fixing slot (241), the second face (2412) is closer to the third end (21), and the first face (2411) is closer to the fourth end (22).

6. The liquid flow sleeve according to claim 4 or 5, wherein both the first face (2411) and the second face (2412) form an angle greater than 30 degrees with the first direction X.

7. The liquid flow sleeve according to any one of claims 3 to 6, wherein an extension direction of the connecting shaft (44) is perpendicular to the first direction X.

8. The liquid flow sleeve according to any one of claims 3 to 7, wherein the extension direction of the connecting shaft (44) is parallel to the first direction X, and a side wall of the sleeve body (10) has a fixing through hole (15) for the fixing protrusion (41) to extend into an inner cavity of the sleeve body (10).

9. The liquid flow sleeve according to any one of claims 2 to 8, wherein the adjustment portion (24) comprises a first arc-shaped adjustment plate (242), the first arc-shaped adjustment plate (242) extending in the first direction X, one end of the first arc-shaped adjustment plate (242) in the first direction X being connected to the adjustment sleeve (23), and the other end of the first arc-shaped adjustment plate (242) in the first direction X being a free end; or
the adjustment portion (24) comprises a connecting tube (243) and a plurality of second arc-shaped adjustment plates (244), the plurality of second arc-shaped adjustment plates (244) extending in the first direction X, one end of the plurality of second arc-shaped adjustment plates (244) in the first direction X being connected to the adjustment sleeve (23), the other end of the plurality of second arc-shaped adjustment plates (244) in the first direction X being connected to the connecting tube (243), the plurality of second arc-shaped adjustment plates (244) being spaced along a circumference of the adjustment sleeve (23), and the fixing slots (241) being located in at least one second arc-shaped adjustment plate (244) of the plurality of second arc-shaped adjustment plates (244); or
the adjustment portion (24) comprises a cylindrical tube (245), the cylindrical tube (245) extending in the first direction X.

10. The liquid flow sleeve according to any one of claims 2 to 9, wherein the outer side wall of the adjustment portion (24) has a limiting protrusion (246), the limiting protrusion (246) and the plurality of fixing slots (241) being arranged in the first direction X, and the limiting protrusion (246) being located on a side of the plurality of fixing slots (241) close to the fourth end (22).

11. The liquid flow sleeve according to any one of claims 2 to 10, wherein the inner side wall of the sleeve body (10) has a first planar face (16), the first planar face (16) being parallel to the first direction X, and the first planar face (16) extending to an end face of the second end (12); the outer side wall of the adjustment sleeve (23) has a second planar face (231), the second planar face (231) extending to an end face of the other end of the adjustment sleeve (23) opposite the third end (21); and the first planar face (16) fits against the second planar face (231).

12. The liquid flow sleeve according to claim 11, wherein the inner side wall of the sleeve body (10) has a first boss (17) protruding into the inner cavity of the sleeve body (10), and a surface of the first boss (17) facing the inner cavity of the sleeve body (10) is the first planar face (16);
in a direction perpendicular to the second planar face (231), a height H1 of the other end of the adjustment sleeve (23) opposite the third end (21) is less than a height H2 of the third end (21), such that the third end (21) of the adjustment sleeve (23) forms a second boss (232), the second boss (232) being located on a side of the first boss (17) away from the second end (12); and
an orthographic projection of the first boss (17) on a first surface at least partially overlaps with an orthographic projection of the second boss (232) on the first surface, the first surface being perpendicular to the first direction X.

13. The liquid flow sleeve according to any one of claims 1 to 12, wherein the mounting assembly (30) is sleeved on the first end (11) of the sleeve body (10), and the outer side wall of the sleeve body (10) has a circle of mounting groove (18) at the first end (11), the mounting groove (18) surrounding the sleeve body (10) along a circumference of the sleeve body (10); and
the mounting assembly (30) has a circle of first annular boss (31) on the inner side wall of an end facing the sleeve body (10), the first annular boss (31) being located in the mounting groove (18).

14. The liquid flow sleeve according to claim 13, wherein the outer side wall of the sleeve body (10) has a second annular boss (19) and a third annular boss (110) spaced in the first direction X, and the mounting groove (18) is formed between the second annular boss (19) and the third annular boss (110).

15. The liquid flow sleeve according to any one of claims 2 to 12, wherein one of the inner side wall of the sleeve body (10) and the outer side wall of the adjustment sleeve (23) has a guide groove (50), the guide groove (50) extending in the first direction X; and the other of the inner side wall of the sleeve body (10) and the outer side wall of the adjustment sleeve (23) has a guide protrusion (60), the guide protrusion (60) being movably located within the guide groove (50).

16. The liquid flow sleeve according to any one of claims 1 to 15, wherein the adjustment assembly (20) comprises a plurality of sub-adjustment assemblies (201), the plurality of sub-adjustment assemblies (201) being connected by means of the connecting assembly (40), and any two adjacent sub-adjustment assemblies (201) being movably connected in the first direction X.

17. A medical ultrasonic scalpel, comprising:
a liquid flow sleeve (100) according to any one of claims 1 to 16;
a handle (200) connected to the mounting assembly (30) of the liquid flow sleeve (100); and
a blade (300) connected to the handle (200), the blade (300) and the liquid flow sleeve (100) being both connected to a same end of the handle (200), and the liquid flow sleeve (100) being sleeved on the blade (300).

18. A robot-assisted ultrasonic scalpel system, comprising a robot-assisted surgical device, and a medical ultrasonic scalpel according to claim 17;
wherein the robot-assisted surgical device is connected to a blade of the medical ultrasonic scalpel to control the movement of the blade.
